# EUROPEAN PATENT SPECIFICATION

(11) **EP 2 952 896 B1**
(45) Date of publication and mention of the grant of the patent: **28.11.2018**
(21) Application number: 14745451.6
(22) Date of filing: 31.01.2014
(51) Int. Cl.: G01N 33/543, G01N 33/53

(54) **METHOD FOR REDUCING FALSE NEGATIVES IN IMMUNOASSAY FOR ASSAYING BIOMEMBRANE-DERIVED SPECIMEN**
VERFAHREN ZUR REDUZIERUNG VON FALSCHEN NEGATIVEN IN EINEM IMMUNOASSAY ZUR BESTIMMUNG VON AUS BIOMEMBRANEN ABGELEITETEN PROBEN
PROCÉDÉ DE RÉDUCTION DE FAUX NÉGATIFS DE DOSAGE IMMUNOLOGIQUE D'ANALYSE DE SPÉCIMEN DÉRIVÉ D'UNE BIOMEMBRANE

(30) Priority: 31.01.2013 JP 2013017134
(43) Date of publication of application: 09.12.2015
(73) Proprietor: Denka Seiken Co., Ltd., Chuo-ku Tokyo 103-8338 (JP)
(72) Inventor: MIYAZAWA, Takashi, Gosen-shi Niigata 959-1695 (JP); SHINOHARA, Yuki, Gosen-shi Niigata 959-1695 (JP)
(74) Representative: Wohlfahrt, Jan Günther
(86) International application number: PCT/JP2014/052254
(87) International publication number: WO 2014/119725

(56) References cited:
- EP-A1- 0 064 274
- EP-A1- 2 602 619
- JP-A- H10 185 920
- JP-A- 2005 241 415
- JP-A- 2012 037 253
- JP-B2- S6 367 864
- US-A1- 2004 018 556
- SMEENK R ET AL: "Influence of pH on the detection of low- and high-avidity anti-dsDNA", JOURNAL OF IMMUNOLOGICAL METHODS, ELSEVIER SCIENCE PUBLISHERS B.V.,AMSTERDAM, NL, vol. 55, no. 3, 30 December 1982 (1982-12-30), pages 361-373, XP023895973, ISSN: 0022-1759, DOI: 10.1016/0022-1759(82)90096-5 [retrieved on 1982-12-30]

## Description

### Technical Field

The present invention relates to a composition of a specimen treatment solution for accurately and specifically detecting a target substance in a specimen in immunoassay using a specimen derived from a biological mucous membrane and a pretreatment method.

### Background Art

As a method for detecting a target substance in a specimen derived from a living body, Enzyme Linked Immuno Assay, immunochromatography and latex turbidimetry by an autoanalyzer are mentioned, for example. As the specimens to be used in these methods, blood, serum, sputum, saliva, pharynx swab, nasal swab, nasal aspirate and keratoconjunctive swab specimen are mentioned, for example.

Most of these specimens except blood and serum are derived from a biological mucous membranes and contain many host defense components derived from the mucous-membrane immune system. It is known that these components are the causes for false positive and false negative in detecting target substances by immunoassay (see, for example, Patent Literature 1).

Many of rapid diagnostics principally based on immunochromatography have been widely used as a means for rapidly and simply detecting infectious diseases caused by viruses or bacteria and determining therapeutic strategy.

Patent literature 2 discloses an immunochromatography reagent composition. Patent literature 3 discloses reagents and methods for the suppression of false positive and false negative results. Patent literature 4 relates to the problem that body fluids have a wide variety of compositions and teaches the treatment of a sample with a polyamine for preventing nonspecific reactions. Non-patent literature 1 pertains to the influence of pH on DNA-binding antibodies.

The false positive and false negative upon the use of rapid diagnostics principally based on immunochromatography lead to wrong therapies, delay treatments and sometimes cause patients to die. In particular, a false negative is an extremely significant problem since it interferes with accurate diagnoses and therapies.

### Citation List

### Patent Literature

Patent Literature 1: JP Patent Publication (Kokai) No. 2009-52945
Patent literature 2: EP 2602619 A1
Patent literature 3: US 2004/0018556 A1
Patent literature 4: EP 0064274 A1
Non-patent literature 1: Smeenk R. et al.; J. Immunol. Methods 55 (1982), pages 361-373

### Summary of Invention

### Technical Problem

The problem to be solved is accurately and specifically detecting a target substance by suppressing the action of an immunoassay inhibitory substance contained in a specimen derived from a biological mucous membrane.

### Solution to Problem

The present inventors have found that a target substance can be accurately and specifically detected by adding a molecule having two or more sulfate groups to a solution for treating a specimen derived from a biological mucous membrane and arrived at the present invention.

The present invention is more specifically as follows.
[1] A method for suppressing a false negative in an immunoassay for analyzing a target substance in a specimen derived from a biological mucous membrane, said method including treating a specimen with a specimen treatment solution containing PIPES which is a compound having two or more sulfate groups.
[2] The method according to [1], in which the specimen derived from a biological mucous membrane is a specimen selected from sputum, saliva, pharynx swab, nasal swab, nasal aspirate, keratoconjunctive swab and feces.
[3] The method according to [1] or [2], in which the compound having two or more sulfate groups is according to the invention PIPES (piperazine-1,4-bis(2-ethanesulfonic acid)) or not being part of the invention a dextran sulfate.
[4] Disclosed is also that the dextran sulfate is dextran sulfate sodium having an average molecular weight of 5,000 to 500,000.
[5] The method according to any one of [1] to [4], in which the compound having two or more sulfate groups is contained in a concentration of 0.1 to 2 (w/v)% in the specimen treatment solution.
[6] The method according to any one of [1] to [5], in which immunoassay is immunochromatography.

The present specification refers to the contents described in the specification and drawings of Japanese Patent Application No. 2013-017134 based on which the priority of the present application is claimed.

### Advantageous Effects of Invention

A false negative in immunoassay using a specimen derived from a biological mucous membrane can be suppressed by treating the specimen derived from a biological mucous membrane with a specimen treatment solution containing a compound having two or more sulfate groups, to enable accurate detection of a target substance.

### Description of Embodiments

Now, the present invention will be more specifically described below.

The present invention relates to a method for accurately analyzing a target substance in immunoassay while preventing a false positive and a false negative, in particular a false negative. The false positive herein refers to the case where a signal indicating a positive is generated in an assay although a target substance is not contained in a specimen; whereas the false negative refers to the case where no signal indicating a positive is generated in an assay although a target substance is contained in a specimen. For example, in immunochromatography, if a target substance is present in a specimen, a complex of a substance immobilized -a target substance-a labelling reagent is formed on a solid-phase substrate, and a signal of a labelling reagent is emitted from the complex; however, in a false negative, generation of a signal is inhibited e.g., for the reason that a complex as mentioned above is not formed.

In the method of the present invention, a subject to be analyzed, i.e., a specimen derived from a biological mucous membrane (mucosal membrane), refers to a specimen contaminated with a substance derived from a biological mucous membrane such as a specimen derived from nasopharynx mucous membrane and a specimen derived from oral cavity mucous membrane. Specific examples thereof may include sputum, saliva, pharynx swab, nasal swab, nasal aspirate, keratoconjunctive swab and feces specimens. The specimen derived from a biological mucous membrane contains host defense components such as IgA involved in the mucous membrane immune system. These components are those which inhibit reactions in immunoassay and cause a false positive and a false negative.

In the method of the present invention, a subject to be analyzed, i.e., a target substance, is an antigen or antibody that can be analyzed by immunoassay, more specifically, an assay using an antigen-antibody reaction. As the antigen, any antigen may be used as long as an antibody can be developed. Examples of the antigen may include proteins, polysaccharides and lipids. Protozoan, fungus, bacteria, mycoplasma, rickettsia, chlamydia, virus, and others containing these substances can be detected.

An assay system for accurately analyzing a target substance while preventing a false positive and a false negative, particularly a false negative in accordance with the method of the invention is immunoassay using an antigen-antibody reaction employing an antibody against a target substance (subject to be analyzed) or employing an antigen binding to an antibody (subject to be analyzed) to be analyzed. Examples of such an analysis system may include ELISA, immunochromatography, an agglutination method, latex turbidimetry by an autoanalyzer, Western blotting and immunoblotting. Of them, immunochromatography susceptible to a substance derived from mucous membrane is preferable.

In the method of the present invention, a specimen derived from a biological mucous membrane is pre-treated with PIPES which is a compound having two or more sulfate groups in a molecule. The pretreatment herein with a compound having two or more sulfate groups in a molecule can be performed by adding a specimen taken from a biological mucous membrane to a specimen treatment solution containing a compound having two or more sulfate groups in a molecule, followed by mixing. In the present invention, a treatment in which a specimen treatment solution (serving as a pretreatment solution) is added to a specimen derived from a biological mucous membrane, followed by mixing to treat a substance to be analyzed contained in the specimen derived from a biological mucous membrane with the specimen treatment solution is referred to as the pretreatment.

As the compound having two or more sulfate groups in a molecule, for example, PIPES (piperazine-1,4-bis(2-ethanesulfonic acid)) and a dextran sulfate such as dextran sulfate sodium (DSS) are mentioned. PIPES is a compound represented by C₈H₁₈N₂O₆S₂ and having two sulfate groups in a molecule. The dextran sulfate sodium is a compound represented by (C₁₁H₁₂O₂₈S₆.6Na)ₙ and having a molecular weight of about 1,000 to 500,000 (average molecular weight: 1,600 to 500,000) although the molecular weight varies depending upon the polymerization degree. As the dextran sulfate sodium, compounds having various average molecular weights are commercially available; for example, compounds having an average molecular weight of about 1,600, an average molecular weight of about 5,000 (molecular weight: 1,000 to 9,000), 6,500 to 10,000, 9,000 to 20,000, about 500,000 are available. The number of sulfate groups varies depending upon the molecular weight. Since the sulfurization degree of dextran sulfate sodium is almost 100%, the number of sulfate groups can be calculated based on the average molecular weight. For example, the number of sulfate groups of a dextran sulfate sodium compound having an average molecular weight of about 5,000 (molecular weight: 1,000 to 9,000) is about 30; the number of sulfate groups in the case of an average molecular weight 6,500 to 10,000 is about 55; the number of sulfate groups in the case of an average molecular weight of 9,000 to 20,000 is about 94 and the number of sulfate groups in the case of an average molecular weight of 500,000 is about 3253. As the compound having two or more sulfate groups in a molecule disclosed, a compound having an average molecular weight of 1,600 to 500,000 or an average molecular weight of 5,000 to 500,000 can be used. As the molecular weight of dextran sulfate sodium increases, a false negative is more effectively suppressed even in a low concentration. For the reason, of the dextran sulfates, dextran sulfate sodium having a molecular weight of 500,000 is preferable.

In the pretreatment according to the method of the present invention, a specimen treatment solution containing PIPES as a compound having two or more sulfate groups in a molecule is added to a specimen derived from a biological mucous membrane as an additive to suspend the specimen in the specimen treatment solution. The specimen treatment solution may contain e.g., a surfactant, BSA (bovine serum albumin) and a salt, other than the compound having two or more sulfate groups in a molecule, in a buffer. Examples of the buffer may include, but not limited to, Tris buffer (about pH6 to 9) and phosphate buffer. The surfactant is not limited and, for example, Triton X-100, Tween 20, Tween 80, Pluronic, sodium dodecyl sulfate (SDS), which are generally used in immunoassay, can be used. Note that, as the compound having two or more sulfate groups in a molecule, a compound having a buffering action can be also used. For example, PIPES as mentioned above is one of the good buffers. Even in this case, a buffer such as Tris buffer may be added as the substance having a buffering action.

The concentration of a compound having two or more sulfate groups in a molecule in a specimen treatment solution is 0.1 to 2 (w/v)%, preferably 0.1 to 1 (w/v)%, more preferably 0.25 to 0.75 (w/v)% and particularly preferably 0.4 to 0.6 (w/v)%.

For example, a specimen as mentioned above may be added to a specimen treatment solution of several hundreds of µL, preferably 200 to 800 µL and stirred well to suspend the specimen. For example, in the case where the specimen is saliva, a cotton swab for sampling a specimen may be impregnated with saliva, soaked in a specimen treatment solution to suspend the specimen. In the case where the specimen is pharynx swab, pharynx mucous membrane may be taken by a cotton swab and the cotton swab is then soaked in a specimen treatment solution to suspend the specimen.

A specimen derived from a biological mucous membrane is suspended in a specimen treatment solution and the specimen treatment solution having the specimen suspended therein is subjected as a test sample to an analysis system using an antigen-antibody reaction to analyze a target substance. After a specimen is mixed with a specimen treatment solution and stirred well to suspend it, the resultant test sample can be immediately used in assay. For example, in immunochromatography, a substance capable of binding to a target substance, such as an antibody, is immobilized onto an appropriate solid-phase substrate such as a nitrocellulose membrane; a complex of the target substance with a substance capable of binding to a target substance and labeled with an appropriate labeling substance (labelling reagent) such as colored polystyrene particles and gold colloid is allowed to migrate and developed on the solid-phase substrate by use of a capillary phenomenon. As a result, a complex of the substance immobilized-the target substance-the labelling reagent is formed on the solid-phase substrate. The target substance can be detected by detecting a signal of the labelling reagent emitted from the complex (in the case of gold colloid, a portion of the solid-phase substrate at which a substance capable of binding to a target substance is bound turns red).

Immunoassay is performed at a temperature of 5 to 35°C, preferably at room temperature. A treatment of a specimen derived from a biological mucous membrane with a specimen treatment solution may be performed at a temperature within this temperature range. The present invention also encompasses a specimen treatment solution containing a compound having two or more sulfate groups in a molecule for suppressing a false negative in immunoassay for analyzing a specimen derived from a biological mucous membrane. The present invention further encompasses an immunoassay kit containing the specimen treatment solution for suppressing a false negative in immunoassay for analyzing a specimen derived from a biological mucous membrane. The immunoassay kit contains, for example, an immunochromatography device containing, for example, a reagent for immunochromatography.

### Examples

The present invention will be more specifically described by way of the following Examples; however, the present invention is not limited to Examples.

### Preparation Example

### 1. Preparation of anti-influenza B virus NP monoclonal antibody

A BALB/c mouse was immunized with an influenza B virus antigen and raised for a predetermined time. The spleen was excised out from the mouse and the spleen cells were fused with mouse myeloma cells (P3 × 63) in accordance with the Kohler's method (Kohler et al., Nature, vol, 256, p495-497 (1975)). The resultant hybridoma cells were kept in an incubator of 37°C. While the antibody activity of the supernatant was checked by ELISA using a plate on which an influenza B virus NP antigen was immobilized, the cells were purified (monoclonalized). Two cell strains were obtained and separately administered intraperitoneally to BALB/c mice treated with pristane. About two weeks later, ascites fluids containing the antibody were collected.

IgG was purified from each of the obtained ascites fluids by affinity chromatography using a protein A column to obtain two types of purified anti-influenza B virus NP antibodies.

### 2. Immobilization of anti-influenza B virus antibody on nitrocellulose membrane

The anti-influenza B virus NP antibody purified was diluted with purified water so as to obtain a concentration of 1.0 mg/mL. The diluted solution was linearly applied to a predetermined position on a nitrocellulose membrane backed with a PET film. The nitrocellulose membrane was dried at 45°C for 30 minutes to obtain an anti-influenza B virus NP antibody immobilized membrane (hereinafter referred to as an antibody immobilized membrane).

### 3. Immobilization of anti-influenza B virus antibody to colored polystyrene particles

Another purified anti-influenza B virus NP antibody, which was not used for immobilization to a nitrocellulose membrane, was diluted with purified water so as to obtain a concentration of 1.0 mg/mL. To this, colored polystyrene particles (manufactured by Thermo scientific) were added so as to obtain a concentration of 0.1 (w/v)%. After stirred, carbodiimide was added to the mixture so as to obtain a concentration of 1 (w/v)% and further stirred. The supernatant was centrifugally removed and the pellet was resuspended in 50 mM Tris (pH9.0) and 3 (w/v)% BSA to obtain anti-influenza B virus NP antibody bound colored polystyrene particles.

### 4. Coating with anti-influenza B virus NP antibody bound colored polystyrene particles, followed by drying

The anti-influenza B virus NP antibody bound colored polystyrene particles obtained in Section 3 were applied to a glass-fiber nonwoven cloth in a predetermined amount of 1.0 µg and dried at 45°C for 30 minutes.

### 5. Adhesion of immobilization membrane to dried pad and other members

Each of the antibody immobilized membrane prepared in Sections 2. and 4. was adhered to a dried pad and other members (backing sheet, absorption band (sample pad)), cut into pieces having a width of 5 mm and used as influenza B virus test strips.

### 6. Preparation of anti-influenza A virus NP monoclonal antibody

A BALB/c mouse was immunized with an influenza A virus antigen and raised for a predetermined time. The spleen was excised out from the mouse and the spleen cells were fused with mouse myeloma cells (P3 × 63) in accordance with the Kohler's method (Kohler et al., Nature, vol, 256, p495-497 (1975)). The resultant hybridoma cells were kept in an incubator of 37°C. While the antibody activity of the supernatant was checked by ELISA using a plate on which an influenza A virus NP antigen was immobilized, the cells were purified (monocloned). Two cell strains were obtained and separately administered intraperitoneally to BALB/c mice treated with pristane. About two weeks later, ascites fluids containing the antibody were collected.

IgG was purified from each of the obtained ascites fluids by use of affinity chromatography using a protein A column to obtain two types of purified anti-influenza A virus NP antibodies.

### 7. Immobilization of anti-influenza A virus antibody virus on nitrocellulose membrane

The anti-influenza A virus NP antibody purified was diluted with purified water so as to obtain a concentration of 1.0 mg/mL. The diluted solution was linearly applied to a predetermined position on a nitrocellulose membrane backed with a PET film. The nitrocellulose membrane was dried at 45 °C for 30 minutes to obtain an anti-influenza A virus NP antibody immobilized membrane (hereinafter referred to as an antibody immobilized membrane).

### 8. Immobilization of anti-influenza A virus antibody to colored polystyrene particles

Another purified anti-influenza B virus NP antibody, which was not used for immobilization to a nitrocellulose membrane, was diluted with purified water so as to obtain a concentration of 1.0 mg/mL. To this, colored polystyrene particles were added so as to obtain a concentration of 0.1 (w/v)%. After stirred, carbodiimide was added to the mixture so as to obtain a concentration of 1 (w/v)% and further stirred. The supernatant was centrifugally removed and the pellet was resuspended in a solution containing 50 mM Tris (pH9.0) and 3 (w/v)% BSA to obtain anti-influenza A virus NP antibody bound colored polystyrene particles.

### 9. Coating with anti-influenza A virus NP antibody bound colored polystyrene particles followed by drying

The anti-influenza A virus NP antibody bound colored polystyrene particles obtained in Section 8 were applied to a glass-fiber nonwoven cloth in a predetermined amount of 1.0 µg and dried at 45°C for 30 minutes.

### 10. Adhesion of immobilization membrane to dried pad and other members

Each of the antibody immobilized membrane prepared in Sections 7. and 9. was adhered to a dried pad and other members such as a backing sheet, an absorption band and a sample pad, cut into pieces having a width of 5 mm and used as influenza A virus test strips. Example 1 Verification of suppression effect of false negative due to saliva specimen in influenza B virus antigen detection immunochromatography

10 mM Tris (pH7.0), 3 (w/v)% BSA and 0.2 (w/v)% Triton X-100 were used as a control specimen treatment solution. Specimen treatment solutions having different compositions were prepared in order to check additives having a sulfate group, more specifically, MES, which is a buffer having a single sulfate group per molecule and PIPES, which is a buffer having two sulfate groups; anionic surfactants, more specifically, sodium dodecyl sulfate (hereinafter referred to as SDS, a single sulfate group/molecule) and sodium deoxycholate (hereinafter referred to as DOC, no sulfate group/molecule); anionic polymers, more specifically, sodium polyacrylate (hereinafter referred to as SPA, no sulfate group/molecule), dextran sulfate sodium (hereinafter referred to as DSS, the number of sulfate groups varies depending upon the dextran molecular weight) and dextran (hereinafter referred to as DEX, no sulfate group/molecule), which was used as a control for dextran sulfate sodium (see Table 1, for details). The additive having a sulfate group was added to a buffer so as to obtain a concentration of 0.5 (w/v)%. Dextran sulfate sodium compounds used in specimen treatment solution Nos. 6 to 9 of Table 1 were obtained from SIGMA (catalog Nos. D8906, D6921, D4911 and D7037, respectively).

A cotton swab (Ex swab-001; manufactured by Denka Seiken Co., Ltd.) was inserted in a mouth and sufficiently impregnated with saliva. The saliva was suspended in each of the specimen treatment solutions (400 µL) having different compositions. To the suspension solution, inactivated influenza B virus (30 µL) was added and mixed. The resultant solution mixture (50 µL) was added dropwise on the sample pads of the influenza virus B test strips. Ten minutes later, determination was visually performed. A result where a signal was observed on a B-type test line was evaluated as "+", a result where a signal was clearly weak as "+ weak", a result where determination of a signal was not easily made as "±", and a result where a signal was not detected as "-". Simultaneously, specimen treatment solutions containing no saliva were subjected to the test. Effects of the compositions of the treatment solutions on the reaction system were checked.

**[Table 1]**

| Composition of specimen treatment solution and number of sulfate groups per molecule to be checked | | | |
|---|---|---|---|
| Specimen treatment solution No. | Composition of specimen treatment solution | Average molecular weight of molecule to be checked | Number of sulfate groups per molecule to be checked |
| Control | 10mM Tris(pH7.0), 3%BSA, 0.2%Triton X-100 | - | None |
| 1 | 10mM MES(pH7.0), 3%BSA, 0.2%TritonX-100 | 213.25 | 1 |
| 2 | 10mM PIEPES(pH7.0), 3%BSA, 0.2%TritonX-100 | 353.36 | 2 |
| 3 | 10mM Tris(pH7.0), 3%BSA, 0.2%Triton X-100, 0.5%SDS | 288.38 | 1 |
| 4 | 10mM Tris(pH7.0), 3%BSA, 0.2%Triton X-100, 0.5%DOC | - | 0 |
| 5 | 10mM Tris(pH7.0), 3%BSA, 0.2%Triton X-100, 0.5%SPA(sodium polyacrylate) | - | 0 |
| 6 | 10mM Tris(pH7.0), 3%BSA, 0.2%Triton X-100, 0.5%DSS | 500,000 | About 3253 |
| 7 | 10mM Tris(pH7.0), 3%BSA, 0.2%Triton X-100, 0.5%DSS | 9,000∼ 20,000 | About 94 |
| 8 | 10mM Tris(pH7.0), 3%BSA, 0.2%Triton X-100, 0.5%DSS | 6,500∼ 10,000 | About 55 |
| 9 | 10mM Tris(pH7.0), 3%BSA, 0.2%Triton X-100, 0.5%DSS | 5,000 | About 30 |
| 10 | 10mM Tris(pH7.0), 3%BSA, 0.2%Triton X-100, 0.5%DEX | 15000∼ 20000 | 0 |

As a result, the number of false negative results in control was increased by adding saliva. In contrast, MES and PIPES, which are buffers having a sulfate group(s), suppressed the number of false negative results from increasing. In particular, the effect of PIPES having two sulfate groups was high (according to the invention). In the system of anionic surfactants to which saliva was not added, it was found that the reaction tends to be inhibited; whereas the number of false negative were slightly reduced in the system containing SDS but a significant decrease of sensitivity was observed. There was a possibility that these surfactants already removed the antibody immobilized to the nitrocellulose membrane or denatured an influenza B virus NP antigen or an anti-influenza B virus NP antibody by the denaturation effects they had. In the anionic polymers, no effect was produced by a sodium polyacrylate having a carboxyl group; whereas, false negative can be almost completely suppressed from increasing by dextran sulfate sodium having sulfate groups. No effect was produced by dextran serving as a control for dextran sulfate sodium.

From the above results, it was demonstrated that an increase of false negative by a saliva specimen can be suppressed by adding a molecule having two or more sulfate groups to a treatment solution. Although data are not shown herein, as the molecular weight of dextran sulfate sodium increases, false negative can be suppressed even in a low concentration and low molecular weight dextran sulfate sodium had to be used in a high concentration. For the reason, on and after Example 2, verification was performed by use of dextran sulfate sodium having an average molecular weight of 500,000.

The results obtained are shown in Table 2.

**[Table 2]**

| Suppression effect of molecule having a sulfate group on false negative due to saliva specimen (influenza B virus) | | |
|---|---|---|
| Specimen treatment solution No. | Without saliva | With saliva |
| Control | + | - |
| 1 | + | ± |
| 2 | + | +weak |
| 3 | ± | ±* |
| 4 | + | - |
| 5 | + | - |
| 6 | + | + |
| 7 | + | + |
| 8 | + | + |
| 9 | + | + |
| 10 | + | - |

| | | |
|---|---|---|
| * Weaker than a sample without saliva | | |

### Example 2 (not according to the invention) Verification of suppression effect on false negative due to a saliva specimen in influenza A virus antigen detection immunochromatography

Suppression effect on false negative due to saliva in influenza A virus antigen detection immunochromatography was checked by using specimen treatment solution No. 6, which showed the highest effect in Example 1.

A cotton swab (Ex swab-001; manufactured by Denka Seiken Co., Ltd.) was inserted in a mouth and sufficiently impregnated with saliva. The saliva was suspended in each of the specimen treatment solutions (400 µL) having different compositions. To the suspension solution, inactivated influenza A virus (30 µL) was added and mixed. The resultant solution mixture (50 µL) was added dropwise on the sample pads of the influenza A virus test strips. Ten minutes later, determination was visually performed.

As a result, the false negative due to saliva were successfully suppressed by using specimen treatment solution No. 6, in the same as in Example 1 (summarized in Table 3).

**[Table 3]**

| Suppression effect of molecule having sulfate groups on false negative due to saliva specimen (influenza A virus) | | |
|---|---|---|
| Specimen treatment solution No. | Without saliva | With saliva |
| Control | + | - |
| 6 | + | + |

### Example 3 (not according to the invention) Verification of suppression effect of false negative due to nasopharynx swab specimen in influenza A and B virus antigen detection immunochromatography

Suppression effect on false negative due to nasopharynx swab specimen in influenza A and B virus antigen detection immunochromatography was checked by using specimen treatment solution No. 6, which showed the highest effect in Example 1.

Nasopharynx swab specimen was collected by use of a cotton swab (Ex swab-002; manufactured by Denka Seiken Co., Ltd.) and suspended in each of the specimen treatment solutions (400 µL) having different compositions. To the suspension solution, inactivated influenza A virus or inactivated influenza B virus (30 µL) was added and mixed. The resultant solution mixture (50 µL) was added dropwise on the sample pads of each of the test strips. Ten minutes later, determination was visually performed.

As a result, false negative due to nasopharynx swab specimen were successfully suppressed by using specimen treatment solution No. 6 in the same as in Examples 1 and 2 (summarized in Table 4).

**[Table 4]**

| Suppression effect of molecule having sulfate group(s) on false negative due to nasopharynx swab specimen | | | | |
|---|---|---|---|---|
| (Influenza A virus or B) | | | | |
| Specimen treatment solution No. | Influenza A virus without nasopharynx swab | Influenza A virus with nasopharynx swab | Influenza B virus without nasopharynx swab | Influenza B virus with nasopharynx swab |
| Control | + | - | + | - |
| 6 | + | + | + | + |

From the above results, it was found that false negative in immunoassay due to specimens derived from nasopharynx mucous membrane and a saliva specimen (a specimen derived from oral cavity mucous membrane) can be suppressed by use of the specimen treatment solution containing a molecule having a sulfate group (particularly, a polymer having two or more sulfate groups).

### Industrial Applicability

The present invention made it possible to construct an immunoassay system that can provide accurate analysis while suppressing a false negative.

## Claims

1. A method for suppressing a false negative in an immunoassay for analyzing a target substance in a specimen derived from a biological mucous membrane, said method comprising treating a specimen with a specimen treatment solution containing PIPES (piperazine-1,4-bis(2-ethanesulfonic acid)) which is a compound having two or more sulfate.

2. The method according to Claim 1, wherein the specimen derived from a biological mucous membrane is a specimen selected from sputum, saliva, pharynx swab, nasal swab, nasal aspirate, keratoconjunctive swab and feces.

3. The method according to Claim 1 or 2, wherein PIPES (piperazine-1,4-bis(2-ethanesulfonic acid)) is comprised in a concentration of 0.1 to 2 (w/v)% in the specimen treatment solution.

4. The method according to any one of Claims 1 to 3, wherein the immunoassay is immunochromatography.

## Patentansprüche

1. Verfahren zum Unterdrücken eines falschen Negativs in einem Immunassay zum Analysieren einer Zielsubstanz in einer Probe, die aus einer biologischen Schleimmembran gewonnen wurde, wobei das Verfahren das Behandeln einer Probe mit einer Probenbehandlungslösung umfasst, die PIPES (Piperazin-1,4-bis(2-ethansulfonsäure)) enthält, die eine Verbindung ist, die zwei oder mehr Sulfate aufweist.

2. Verfahren nach Anspruch 1, wobei die aus einer biologischen Schleimmembran gewonnene Probe eine Probe ist, die ausgewählt ist aus Sputum, Speichel, Rachenabstrich, Nasenabstrich, Nasenaspirat, Horn- und Bindehautabstrich und Fäkalien.

3. Verfahren nach Anspruch 1 oder 2, wobei PIPES (Piperazin-1,4-bis(2-ethansulfonsäure)) in einer Konzentration von 0,1 bis 2 (w/v)-% in der Probenbehandlungslösung enthalten ist.

4. Verfahren nach einem der Ansprüche 1 bis 3, wobei der Immunassay Immunchromatografie ist.

## Revendications

1. Procédé permettant de supprimer un faux négatif dans un dosage immunologique destiné à analyser une substance cible dans un prélèvement issu d'une muqueuse biologique, ledit procédé comprenant le traitement d'un prélèvement avec une solution de traitement de prélèvement contenant du PIPES (acide pipérazine-1,4-bis(2-éthanesulfonique)) qui est un composé ayant deux sulfates ou davantage.

2. Procédé selon la revendication 1, dans lequel le prélèvement issu d'une muqueuse biologique est un prélèvement sélectionné parmi du crachat, de la salive, un écouvillon pharyngé, un écouvillon nasal, un aspirat nasal, un écouvillon kérato-conjonctival et des fèces.

3. Procédé selon la revendication 1 ou 2 dans lequel le PIPES (acide pipérazine-1,4-bis(2-éthanesulfonique)) est compris en une concentration de 0,1 à 2 % (pds/v) dans la solution de traitement de prélèvement.

4. Procédé selon l'une quelconque des revendications 1 à 3, dans lequel le dosage immunologique est une immunochromatographie.
